# EUROPEAN PATENT APPLICATION

(11) **EP 4 400 112 A1**
(43) Date of publication of application: **17.07.2024**
(21) Application number: 22867439.6
(22) Date of filing: 09.09.2022
(51) Int. Cl.: A61K 36/062, A61K 8/97, A61P 17/00, A61P 17/02, A61P 43/00, A61Q 19/00, A61Q 19/02, A61Q 19/08

(54) **SKIN COMPOSITION INCLUDING EXTRACELLULAR VESICLES DERIVED FROM MALTED RICE**

(30) Priority: 09.09.2021 JP 2021146860
(71) Applicant: Da Vinci Universale Co., Ltd., Tokyo, 100-0005 (JP); Asahi Shuzo Co., Ltd., Iwakuni-shi, Yamaguchi, 742-0422 (JP)
(72) Inventor: OCHIYA Takahiro, Tokyo 104-0053 (JP); MURANAKA Asao, Kashiwa-shi, Chiba 277-0832 (JP)
(74) Representative: J A Kemp LLP
(86) International application number: PCT/JP2022/033893
(87) International publication number: WO 2023/038118

(57) **Abstract**

A skin composition, comprising an extracellular vesicle derived from koji.

## Description

### Technical Field

The present invention relates to a skin composition comprising a koji-derived extracellular vesicle, in particular, a koji-derived exosome.

### Background Art

Exosome is a membrane vesicle secreted from cells, and the surface of an exosome is composed of a lipid bilayer membrane. The exosome is responsible for local or systemic intercellular information transfer by transporting intracellular proteins and genetic information (mRNA, microRNA, etc.) to the outside of the cells. The functions of the exosome are diverse, and it has been reported that, for example, extracellular vesicles derived from Gram-negative bacteria have anti-cancer action (Non Patent Literature 1: Kim OY et al., Nat Commun, 626, 2017).

It has also been suggested that the functions of the exosome are different depending on the cells from which the exosome is derived. For example, when it is an exosome derived from mesenchymal stem cells such as adipose-derived stem cells, the exosome is known to have the function of suppressing inflammation, the function of treating dementia, etc. (Non Patent Literature 2: Katsuda et al., Methods Mol Biol., 1212, 2015).

Thus, it is considered that exosomes secreted from various types of cells can be used to provide various physiological functions and to treat various diseases.

However, the effects of the exosomes on the skin, for example, on melanocytes in skin, have not been elucidated.

### Citation List

### Non Patent Literature

Non Patent Literature 1: Kim OY et al., Nat Commun, 626, 2017
Non Patent Literature 2: Katsuda et al., Methods Mol Biol., 1212, 2015

### Summary of Invention

### Technical Problem

Under the aforementioned circumstances, it has been desired to develop an exosome useful as an agent for skin.

### Solution to Problem

As a result of intensive studies conducted directed towards achieving the aforementioned object, the present inventor has found that a koji-derived exosome suppresses melanocytes, thereby completing the present invention.

Specifically, the present invention is as follows.
(1) A skin composition, comprising an extracellular vesicle derived from koji.
(2) The composition according to the above (1), wherein the extracellular vesicle is an exosome.
(3) The composition according to the above (1) or (2), wherein the koji is rice koji, barley koji or soybean koji.
(4) The composition according to any one of the above (1) to (3), which is used as a pharmaceutical product, a quasi-drug product or a cosmetic product.
(5) The composition according to any one of the above (1) to (4), which is for use in skin whitening, anti-wrinkle, skin regeneration, skin wound healing, or dermatitis.
(6) A method for producing a koji-derived extracellular vesicle, which is characterized in that it comprises culturing a koji mold, and collecting an extracellular vesicle from the obtained culture.
(7) The method according to the above (6), wherein the koji mold is a yellow koji mold, a white koji mold, a black koji mold, or a red koji mold.
(8) The method according to the above (6) or (7), wherein the extracellular vesicle is an exosome.

### Advantageous Effects of Invention

According to the present invention, an extracellular vesicle derived from koji is provided. Since the extracellular vesicle of the present invention (in particular, a koji-derived exosome) suppresses genes associated with the activity and proliferation of melanocytes and secretion of melanin pigments, and other genes, the present extracellular vesicle is useful as a skin external preparation or a cosmetic product.

### Brief Description of Drawings

[Figure 1] Figure 1 is a view showing the melanocyte-suppressing effect of extracellular vesicles derived from *Dassai* amazake (rice koji).
[Figure 2] Figure 2 is a view showing the results of a melanin synthesis suppression test using B16 cells (Mouse B16 melanoma 4A5).

### Description of Embodiments

### 1. Koji-derived extracellular vesicle

The present invention relates to a skin composition, comprising an extracellular vesicle derived from koji. The composition of the present invention can be used as a composition for pharmaceutical products, quasi-drug products or cosmetic products.

The extracellular vesicle used in the present invention may be any one of an exosome, a microvesicle and an apoptotic body, and it is desirably an exosome. The exosome is an endosome-derived vesicle, the microvesicle is a vesicle directly secreted from cells, and the apoptotic body is a cell fragment generated as a result of cell death.

The method for producing the koji-derived extracellular vesicle of the present invention will be described below, in particular, taking the exosome as an example.

### (1) Koji

Koji is made by propagating microorganisms effective for food fermentation, such as koji molds, on grains such as rice, barley, and soybeans. Therefore, the raw materials for koji include rice, barley, and soybeans.

The above-described raw materials for koji are fermented using a koji mold.

The koji mold used in the present invention is a microorganism that has long been used in the brewing of Japanese *sake*, *miso*, soy sauce, and other products. Since such koji mold has a high ability to secrete and produce proteins, such as several grams of proteins in 1 L of a culture solution, it is also utilized for production of useful proteins such as enzymes.

In the present invention, for example, the following koji molds can be used:
Yellow koji molds: *Aspergillus oryzae*, *Aspergillus sojae*, etc.;
Black koji molds: *Aspergillus nigar*, etc.;
White koji molds: *Aspergillus kawachii*, etc.; and
Red koji molds: molds belonging to genus Monascus.

### (2) Culture

First, the rice is washed with water until white cloudiness disappears. After discarding the washed water, new water is added, and the rice is soaked until it is completely immersed therein. The time of soaking the rice is not particularly limited, and can be adjusted according to the season. For example, the soaking time is for 3 to 5 hours in summer, for 6 to 12 hours in spring and autumn, and for 15 to 20 hours in winter. After the immersion, the rice is drained in a colander for about 2 to 4 hours, and further, is blended once or twice an hour, so that the top and bottom sides of the rice change. Thereafter, the rice is transferred into a steamer and is then steamed. The steamed rice is quickly spread out on a tray or the like that has been sterilized with alcohol (or *shochu*), and the moisture and the heat after steaming are quickly removed from the surface of the rice by such an action as cutting the rice with a rice scoop or the like.

When the temperature of the steamed rice has reached about 36°C, seed malt is sprinkled on the rice. The amount of the seed malt used is, for example, 2 g of the seed malt for 1 kg of the rice. The rice into which the koji is mixed is quickly gathered together, and is then wrapped with a clean cloth.

The rice wrapped in the cloth is kept warm, such that the temperature thereof becomes 30°C to 32°C. Koji is ready at 45 to 48 hours after the spreading of the seed malt. The state in which koji strongly exhibits a chestnut-like flavor, the mycelium grows, and the rice sticks to one another to form a plate, but in which it can be easily loosened, is the approximate state of the finished koji. To this rice koji (raw material), water is added (1 L of water to 100 grams of the rice koji), and is then left at 4°C for 8 to 16 hours to obtain koji water containing extracellular vesicles. Alternatively, leaving the koji overnight at 30°C to 37°C will also produce rice koji containing extracellular vesicles.

### (3) Recovery

Examples of the method of recovering exosomes from cultures (fermented products such as rice koji) may include an ultracentrifugation method, an immunoprecipitation method, an affinity method using magnetic beads, and a density gradient centrifugation method. Commercially available reagents for exosome isolation can also be employed to isolate and recover exosomes.

In the case of the ultracentrifugation method, high-purity exosomes can be obtained, for example, by previously performing centrifugation at a centrifugal force of about 100 g to 10,000 g for several steps, and then performing ultracentrifugation at a centrifugal force of about 120,000 g for 70 minutes. However, those skilled in the art can determine the centrifugation method and conditions, as appropriate.

Whether the recovered exosomes are the intended ones can be confirmed by morphological observation using a transmission electron microscope, the measurement of the number of particles and particle size using a nanoparticle tracking system, a genetic analysis using PCR, an immunological method (ELISA, FACS, etc.), Western blotting, or other methods.

In addition, whether the recovered exosomes are the intended ones can also be confirmed using, as indicators, proteins recognized as exosome markers. For example, CD9, CD63 and the like can be used as markers for detection.

### 2. Skin composition

The present invention relates to a skin composition, comprising an extracellular vesicle derived from koji, in particular, a koji-derived exosome. The skin composition of the present invention is used as a cosmetic product, a pharmaceutical product or a quasi-drug product.

The disorders or diseases serving as subjects, to which the skin composition of the present invention is used, are not particularly limited.

The skin composition of the present invention may include a variety of pharmaceutically (medically) acceptable carriers depending on the form of use, as long as the present skin composition contains an effective amount of exosome serving as an active ingredient, without losing its activity. For example, commonly used carriers can be applied as diluents, excipients, etc.

Examples of such carriers may include water, a normal saline (PBS, etc.), and various organic solvents. Examples of the organic solvents may include an alcohol (ethanol, etc.) aqueous solution, glycerol, and olive oil. Moreover, various fillers, thickeners, binders, surface-active agents, pigments, fragrances, etc. can be added.

When the koji-derived extracellular vesicle of the present invention is used as a pharmaceutical composition, it can be processed in the form of a parenteral preparation such as a liquid agent, a suspending agent, an emulsion, an ointment, an aqueous gel, a sheet or a microneedle, or in the form of an oral preparation such as a granule, a powder agent, a tablet or a capsule.

Moreover, in order to use the present composition for an injection and the like, it can also be processed in a freeze-dried product or a granulated product, which is dissolved in a normal saline, a suitable buffer solution (e.g. PBS), etc. to prepare a drug solution, immediately before the use.

The composition of the present invention can be used in a form, by a method, and at a dosage, which depend on the therapeutic purpose and patient.

The additive amount and the number of additions are predicted to be different depending on the type of skin disease or disorder, as well as conditions such as the culture conditions and the type of a medium necessary for obtaining exosomes. Therefore, those skilled in the art can determine the usage and the dosage, as appropriate.

For example, as an effective amount of exosomes, the concentration of exosomes in the culture medium is 1 × 10⁹ cells/mL or more, and is preferably 5 µg/mL to 20 µg/mL.

The skin composition of the present invention can be produced as each type of composition in accordance with the classification such as a cosmetic product or a quasi-drugs produce (medicated cosmetic product) that is positioned between a cosmetic product and a pharmaceutical product.

Examples of the indications of the skin composition of the present invention may include whitening, anti-wrinkle, skin rash, rashes, heat rash, chilblains, cracks, chapping, acne, oily skin, razor burn, spots/freckles due to sunburn, burning after sunburn or snow burn, skin tightening, skin cleaning, skin conditioning, keeping skin healthy, moisturizing skin, skin protection, prevention of skin from drying out, and reduction in fine lines and wrinkles caused by dryness. Moreover, the skin composition of the present invention can be used for skin cancer, skin trauma, burns, skin regenerative medicine, skin wound healing, atopic dermatitis, etc.

Preferred examples of the indications of the present skin composition may include whitening, anti-wrinkle, skin regeneration, skin wound healing, and atopic dermatitis.

Examples of the dosage form of the skin composition of the present invention may include a liquid agent, a spray agent, an ointment, a cream agent, a gelling agent, and a patch. In addition, when the present skin composition is used as a skin external preparation or a cosmetic product, examples thereof may include basic cosmetic products (lotion, cream, emulsion, serum, facial cleanser, etc.), make-up cosmetic products (foundation, etc.), hair care products (shampoo, conditioner, hairdressing, etc.), other cosmetics (a whitening agent, a hair remover, etc.).

Moreover, as described above, the present skin composition may also be in the form of an oral preparation such as a granule, a powder agent, a tablet or a capsule.

The skin composition of the present invention may comprise ingredients other than exosomes, depending on its classification (a cosmetic product, a quasi-drug product, etc.) and its dosage form. Examples of the ingredients other than exosomes may include additives commonly used in production of cosmetic products or quasi-drug products (in particular, skin external preparations and compositions for transdermal administration), such as a stabilizer, a preservative, a buffer, a pH adjuster, and an excipient. The contents of such ingredients other than exosomes can also be controlled, as appropriate, by those skilled in the art.

As necessary, it is also possible to use drugs that are known to be mixed into cosmetic products, quasi-drug products, etc., not as ingredients that are to be encapsulated in exosomes, but as ingredients that to be mixed into skin external preparations in a state in which the ingredients are not encapsulated in exosomes. Examples of such drugs may include vitamins A, C, B and E and their derivatives, and estrogens.

### Examples

Hereinafter, the present invention will be more specifically described in the following Examples. However, these Examples are not intended to limit the scope of the present invention.

### [Example 1]

### (1) Fluctuation in gene expression of melanocytes by extracellular vesicle derived from rice koji

Normal human epidermal melanocytes (Human Epidermal Melanocyte, Normal, Cryopreserved <NHEM-c>, Funakoshi Co., Ltd., C-12400, origin: underage foreskin) were used as human melanocytes.

The melanocytes were cultured using Melanocyte Growth Medium Kit (Funakoshi Co., Ltd., C-24110), and the culture was carried out in accordance with the procedures included therewith.

Using T-25 (Nunc EasYFlask156367 that had been treated for cell culture) as a culture flask, the cells were allowed to proliferate to 80% confluency, and extracellular vesicles derived from rice koji were then added to the cells. The concentration of the extracellular vesicles added was 100 to 1000 extracellular vesicles per cell.

After the extracellular vesicles derived from rice koji had been added to the cell culture medium, the cells were cultured in a CO₂ incubator for 72 hours. After completion of the culture, the cells were recovered using a cell scraper, and mRNA was then recovered using the mRNA purification kit of Qiagen (RNAeasy mini kit).

Based on this mRNA, the expression levels of the genes Tyrosinase, ROS, EPR, ETBR and Mc1r were quantified by quantitative PCR (QuantiTect Probe PCR Kit (200), Qiagen). The relative expression levels of individual genes after 72 hours were calculated using the expression levels of individual genes in the cells before addition of the extracellular vesicles as 100.

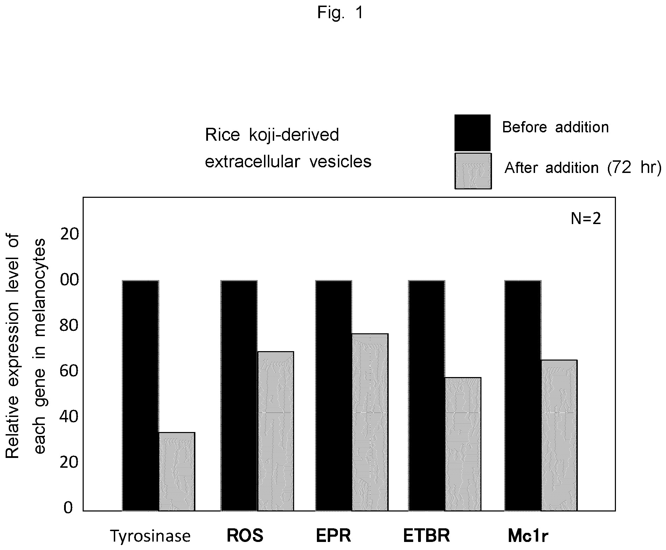

As a result, it was found that the expression of all melanin synthesis-related genes was suppressed (Figure 1). The mean value of N = 2 was shown in the figure.

### (2) Melanin synthesis suppression test using B16 cells (Mouse B16 melanoma 4A5)

As cells, the mouse-derived B16 melanoma 4A5 cell line (Merck: 94042254) was used.

As a medium, the Gibco DMEM + 2 mM glutamine + 10% fetal bovine serum (FBS) was used.

The B16 cells were cultured in a 6-well plate (Corning), and when the cells became 85% confluency, extracellular vesicles derived from rice koji were added thereto.

The concentration of the extracellular vesicles added was 100 to 1000 extracellular vesicles per cell, and a comparison was made in this range. After the extracellular vesicles had been added to the cell culture medium, the cells were cultured in a CO₂ incubator for 72 hours. After completion of the culture, melanin contained in the cells was extracted (dissolved in 1 M NaOH (Wako Pure Chemical Industries, Ltd.)), and the absorbance was then measured (OD 420 nm) using an absorption spectrometer. The measurement results were expressed as relative values with the amount of melanin in untreated B16 cells as 100. The mean value of N = 3 was shown.

As a control group, a PBS(-) and vitamin C (10 mg/ml) addition group was used.

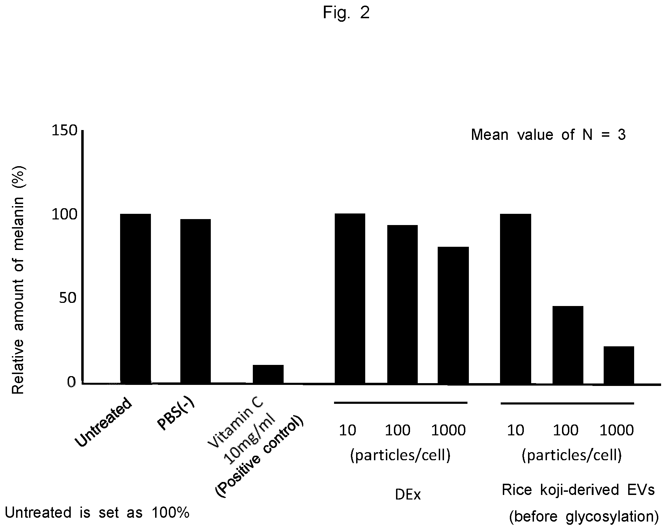

As a result, when the extracellular vesicles derived from rice koji were added, the amount of melanin pigment was attenuated in the dose-dependent manner (Figure 2).

## Claims

1. A skin composition, comprising an extracellular vesicle derived from koji.

2. The composition according to claim 1, wherein the extracellular vesicle is an exosome.

3. The composition according to claim 1 or 2, wherein the koji is rice koji, barley koji or soybean koji.

4. The composition according to any one of claims 1 to 3, which is used as a pharmaceutical product, a quasi-drug product or a cosmetic product.

5. The composition according to any one of claims 1 to 4, which is for use in skin whitening, anti-wrinkle, skin regeneration, skin wound healing, or dermatitis.

6. A method for producing a koji-derived extracellular vesicle, which is **characterized in that** it comprises culturing a koji mold, and collecting an extracellular vesicle from the obtained culture.

7. The method according to claim 6, wherein the koji mold is a yellow koji mold, a white koji mold, a black koji mold, or a red koji mold.

8. The method according to claim 6 or 7, wherein the extracellular vesicle is an exosome.
